# EUROPEAN PATENT APPLICATION

(11) **EP 3 095 404 A1**
(43) Date of publication of application: **23.11.2016**
(21) Application number: 16169870.9
(22) Date of filing: 17.05.2016
(51) Int. Cl.: A61B 18/14

(54) **CATHETER WITH ANCHORING BALLOON ASSEMBLY**

(30) Priority: 18.05.2015 US 201514715133
(71) Applicant: Biosense Webster (Israel) Ltd., 2066717 Yokneam (IL)
(72) Inventor: O'FALLON, Patrick, Toluca Lake, CA 91602 (US); DITTER, Tom Allen, Mission Viejo, CA 92692 (US); MASSOUD, Hamid, Los Angeles, CA 90026 (US)
(74) Representative: Carpmaels & Ransford LLP

(57) **Abstract**

A catheter has an electrode assembly adapted for use on an ostium and a stabilizing assembly movable between a stowed position within the catheter and a deployed position in the tubular region of the ostium. The stabilizing assembly has a balloon member that can be extended distally into the tubular region and inflated to wedge itself in the tubular region for anchoring the electrode assembly. The balloon member is mounted on an elongated extender that extends through the catheter and is provided with longitudinal movement to advance and withdraw the balloon between the stowed and deployed positions.

## Description

### FIELD OF INVENTION

The present invention relates to an improved electrophysiologic catheter that is particularly useful for use in or near a tubular region of the heart.

### BACKGROUND OF INVENTION

Cardiac arrythmias, and atrial fibrillation in particular, persist as common and dangerous medical ailments, especially in the aging population. In patients with normal sinus rhythm, the heart, which is comprised of atrial, ventricular, and excitatory conduction tissue, is electrically excited to beat in a synchronous, patterned fashion. In patients with cardiac arrythmias, abnormal regions of cardiac tissue do not follow the synchronous beating cycle associated with normally conductive tissue as in patients with normal sinus rhythm. Instead, the abnormal regions of cardiac tissue aberrantly conduct to adjacent tissue, thereby disrupting the cardiac cycle into an asynchronous cardiac rhythm. Such abnormal conduction has been previously known to occur at various regions of the heart, such as, for example, in the region of the sino-atrial (SA) node, along the conduction pathways of the atrioventricular (AV) node and the Bundle of His, or in the cardiac muscle tissue forming the walls of the ventricular and atrial cardiac chambers.

Cardiac arrhythmias, including atrial arrhythmias, may be of a multiwavelet reentrant type, characterized by multiple asynchronous loops of electrical impulses that are scattered about the atrial chamber and are often self propagating. Alternatively, or in addition to the multiwavelet reenetrant type, cardiac arrhythmias may also have a focal origin, such as when an isolated region of tissue in an atrium fires autonomously in a rapid, repetitive fashion.

A host of clinical conditions may result from the irregular cardiac function and resulting hemodynamic abnormalities associated with atrial fibrillation, including stroke, heart failure, and other thromboembolic events. In fact, atrial fibrillation is believed to be a significant cause of cerebral stroke, wherein the abnormal hemodynamics in the left atrium caused by the fibrillatory wall motion precipitate the formation of thrombus within the atrial chamber. A thromboembolism is ultimately dislodged into the left ventricle, which thereafter pumps the embolism into the cerebral circulation where a stroke results. Accordingly, numerous procedures for treating atrial arrhythmias have been developed, including pharmacological, surgical, and catheter ablation procedures.

It has been found that by mapping the electrical properties of the endocardium and the heart volume, and selectively ablating cardiac tissue by application of energy, it is sometimes possible to cease or modify the propagation of unwanted electrical signals from one portion of the heart to another. The ablation process destroys the unwanted electrical pathways by formation of non-conducting lesions. Examples of catheter-based devices and treatment methods have generally targeted atrial segmentation with ablation catheter devices and methods adapted to form linear or curvilinear lesions in the wall tissue which defines the atrial chambers, such as those disclosed in U.S. Patent No 5,617,854 to Munsif, U.S. Patent No. 4,898,591 to Jang, et al., U.S. Patent No. 5,487,385 to Avitall, and U.S. Patent No. 5,582,609 to Swanson, the disclosures of which are incorporated herein by reference. In addition, various energy delivery modalities have been disclosed for forming such atrial wall lesions, and include use of microwave, laser and more commonly, radiofrequency energies to create conduction blocks along the cardiac tissue wall, as disclosed in WO 93/20767 to Stem, et al., U.S. Patent No. 5,104,393 to Isner, et al. and U.S. Patent No. 5,575,766 to Swartz, et al., respectively, the entire disclosures of which are incorporated herein by reference.

In this two-step procedure--mapping followed by ablation--electrical activity at points in the heart is typically sensed and measured by advancing a catheter containing one or more electrical sensors into the heart, and acquiring data at a multiplicity of points. These data are then utilized to select the target areas at which ablation is to be performed.

Mapping and ablation in regions of or near the pulmonary veins poses special challenges due to the configuration of the ostia and surrounding tubular tissue. Catheters have been developed that are particularly useful for mapping and ablating the pulmonary veins and other tubular regions of or near the heart, including the ostium. Catheters with lasso, basket and balloon assemblies are also known. Such catheters are disclosed in, for example, U.S. Patent Nos., 6,866,662 6,973,339, 7,003,342, 7,142,903, 7,412,273, 8,712,550 the entire disclosures of which are hereby incorporated by reference.

"Lasso" catheters are particularly useful during circumferential ablations around the ostium of the pulmonary veins. However, during a procedure, catheters may dislodge from the ostium with movement of the heart. Accordingly, it is desired that a catheter for use on an ostium or in a tubular region provide an anchoring mechanism to stabilize the catheter in the region of treatment.

### SUMMARY OF THE INVENTION

The present invention is directed to a catheter have an electrode assembly adapted for use on or near an ostium with a stabilizing assembly that can be lodged in the tubular region of the ostium to secure the electrode assembly. The stabilizing assembly may include a balloon member that can be collapsed when stowed or inflated when deployed.

In some embodiments, the catheter comprises an elongated catheter body, a distal section with at least a first lumen and a second lumen, an electrode assembly distal of the distal section having a proximal end received in the first lumen, and a stabilizing assembly longitudinally movable between a stowed position and a deploy positioned, where the stabilizing assembly has a balloon member that is housed in the second lumen of the distal section when the stabilizing assembly is in the stowed position and extends distally of the electrode assembly when the stabilizing assembly is in the deployed position.

In more detailed embodiments, the stabilizing assembly includes an extender having a distal portion on which the balloon member is mounted, and the extender is longitudinally movable relative to at least the distal section of the catheter.

In more detailed embodiments, the extender extends longitudinally through the catheter body and the distal section, and the extender has a distal portion extending through an interior of the balloon member.

In more detailed embodiments, the extender has a lumen therethrough along its length, and the distal portion of the extender has ports configured to allow fluid to pass from the lumen of the extender to the interior of the balloon member to inflate the balloon member.

In more detailed embodiments, the catheter also includes a guide wire tubing for guiding a guide wire. The guide wire tubing may be coaxial with the extender, extending through the lumen of the extender. In other more detailed embodiments, the guide wire tubing extends through the catheter separately from the extender.

In other embodiments, a catheter adapted for use in or near a tubular region of the heart, comprises an elongated catheter body, a distal section having at least a first lumen and a second lumen, a distal electrode assembly having a generally circular main portion configured to contact an ostium of the tubular region, and a balloon assembly longitudinally movable between a stowed position and a deployed position, wherein the balloon is housed in the second lumen in the stowed position, and the balloon is advanced into the tubular region distally of the electrode assembly in the deployed position to stabilize the electrode assembly on the ostium.

In more detailed embodiments, the balloon assembly includes an elongated extender and a balloon member mounted on a distal portion of the extender.

In more detailed embodiments, the extender extends along the length of the catheter and has a proximal portion adapted for manipulation by a user to move the balloon member between the stowed position and the deployed position.

In more detailed embodiment, the extender has a lumen therethough to pass fluid to inflate the balloon member.

In more detailed embodiments, the extender has a sidewall with at least one port through which fluid passes into the balloon member.

In more detailed embodiments, the balloon assembly includes a fluid tubing surrounding the extender, the fluid tubing having a lumen to pass fluid to flush the second lumen before the balloon member is deployed from and/or stowed in the second lumen.

In more detailed embodiments, the catheter also includes a guide wire tubing extending through the catheter, and the guide wire tubing may extend through the balloon assembly, including a lumen of the extender, being coaxial with the extender.

### BRIEF DESCRIPTION OF THE DRAWINGS

These and other features and advantages of the present invention will be better understood by reference to the following detailed description when considered in conjunction with the accompanying drawings wherein:
FIG. 1 is a perspective view of a catheter of the present invention, in accordance with one embodiment.
FIG. 2 is a side view of the catheter of FIG. 1, in a deployed configuration.
FIG. 3A is a side cross-sectional view of the catheter of FIG. 1, including a junction between a catheter body and a distal section.
FIG. 3B is an end cross-sectional view of the catheter body of FIG. 3A, taken along line B-B.
FIG. 4A is a side cross-sectional view of the catheter of FIG. 1, including a distal end of the distal section.
FIG. 4B is an end cross-sectional view of the distal section of FIG. 4A, taken along line B-B.
FIG. 5 is a perspective view of the catheter of FIG. 1, including a distal end of a distal section, a distal electrode assembly and a balloon assembly.
FIG. 6A is a side cross-sectional view of a ring electrode mounted on a distal electrode assembly, in accordance with one embodiment.
FIG. 6B is an end cross-sectional view of the mounted ring electrode of FIG. 6A, taken along line B-B.
FIG. 7A is a perspective view of various connections to the control handle of the catheter, in accordance with one embodiment.
FIG. 7B is a perspective view of various connections to the control handle of the catheter, in accordance with another embodiment.
FIG. 7C is an end cross-sectional view of a connection of FIG. 7B, taken along line C-C.
FIG. 8 is a perspective view of a catheter of the present invention, in accordance with another embodiment, including a distal end of a distal section, a distal electrode assembly and a balloon assembly, with a coaxial guide wire tubing.
FIG. 9A is a side cross-sectional view of the distal end of the distal section of FIG. 8.
FIG. 9B is an end cross-sectional view of the distal section of FIG. 9A, taken along line B-B.

### DETAILED DESCRIPTION OF THE INVENTION

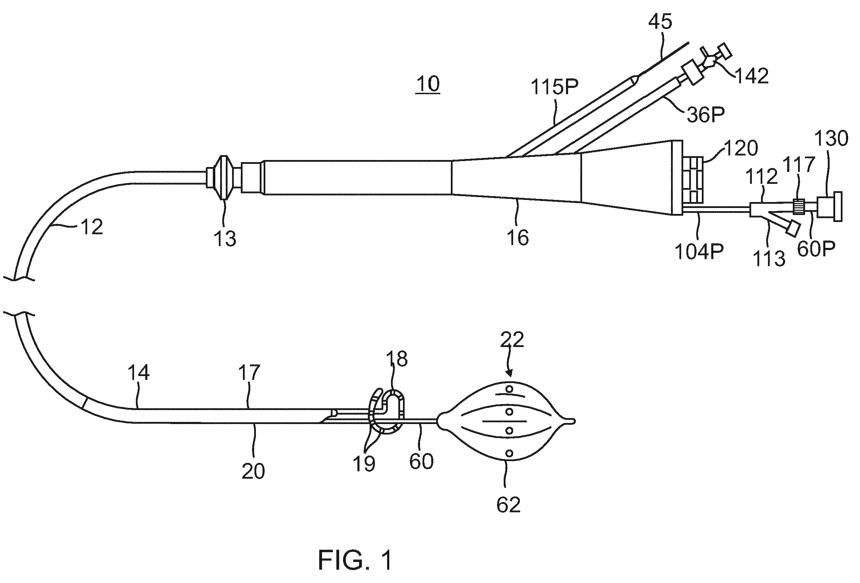

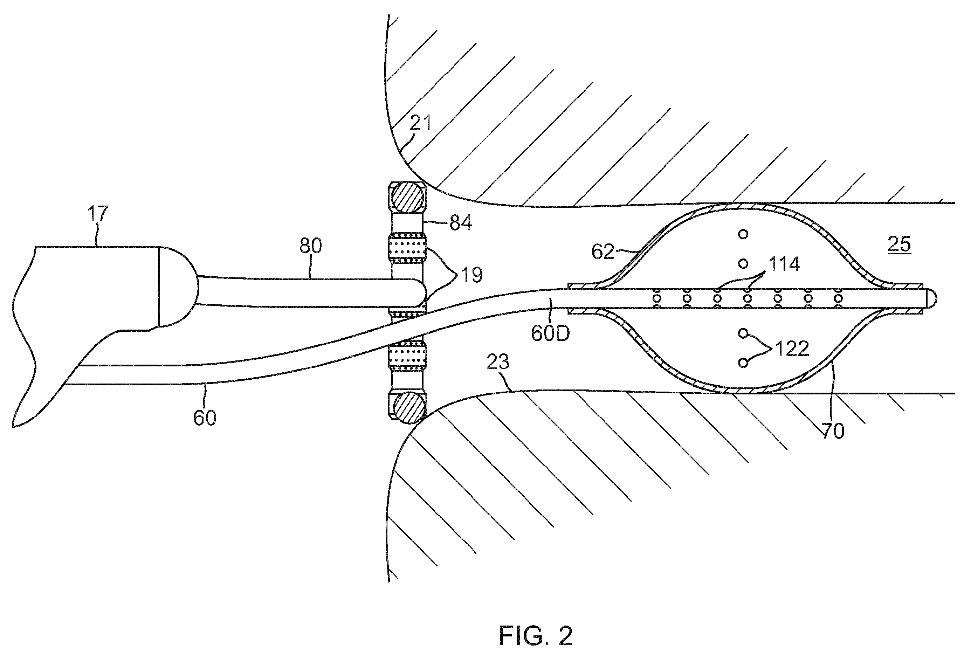

With reference to FIG. 1, embodiments of the present invention provide a catheter 10 having a distal section 17 with an electrode assembly 18 and a nesting portion 20 in which an anchoring/stabilizing assembly 22 may be stowed for deployment into a patient's body in or near a tubular region of the heart to anchor the electrode assembly 18. The catheter comprises an elongated catheter body 12 having proximal and distal ends, a distal section 17 distal of the catheter body, and a control handle 16 proximal of the catheter body 12. In the illustrated embodiment, the electrode assembly 18 is lasso-shaped with a plurality of ring electrodes 19 and adapted to sit on an ostium or opening 21 of a tubular region 25 of the heart, for example, an ostium 21 of a pulmonary vein, for circumferential tissue contact on the ostium, as shown in FIG. 2. The anchoring assembly 22 includes a balloon assembly with an inflatable balloon member 62 and an elongated extender or rod 60 which extends the balloon member 62 past the ostium 21 and into the tubular region 25 for circumferential tissue contact with an inner surface 23 of the tubular region. The balloon member 62 can be sufficiently inflated to wedge the balloon member in the tubular region which stabilizes and anchors the electrode assembly 18 on the ostium. Prior to deployment into the patient's body, the anchoring assembly 22 is nested and stowed in the distal section 17.

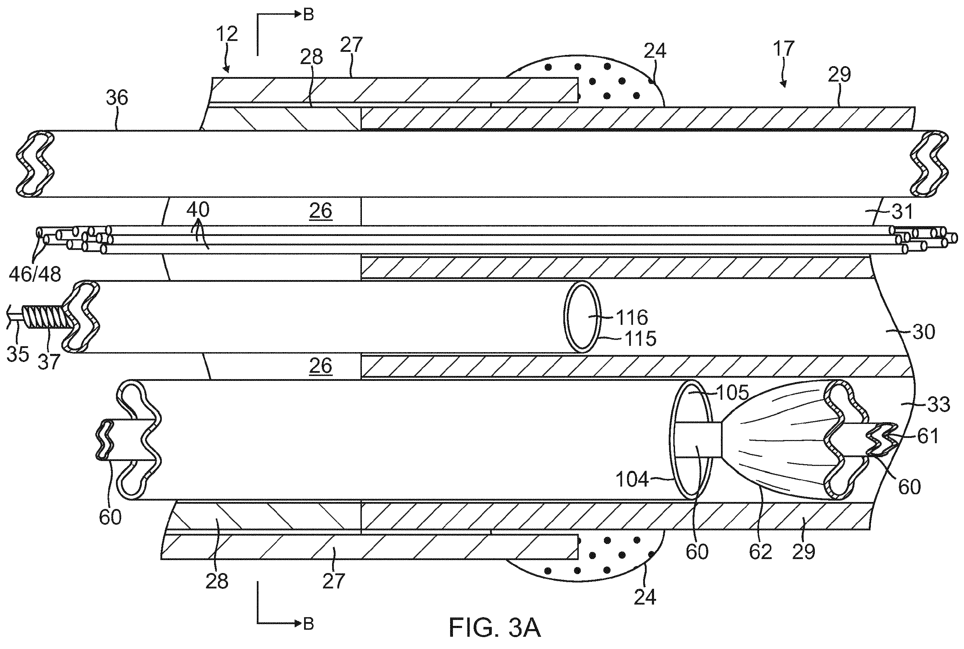

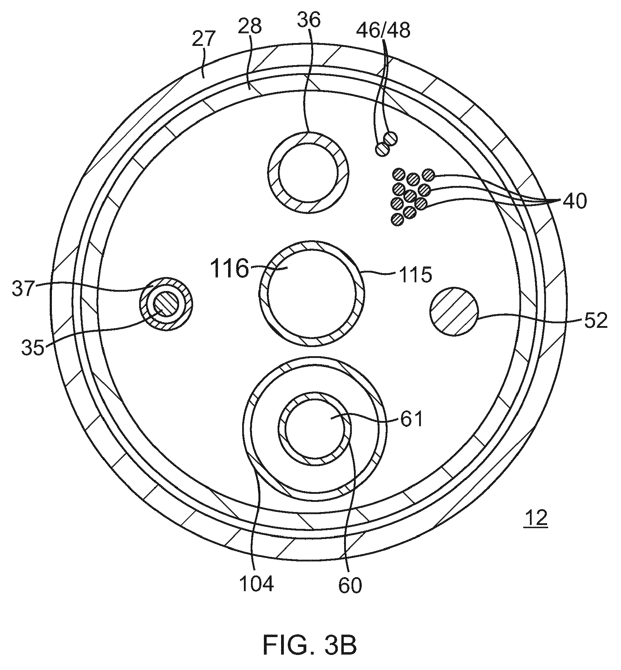

With reference to FIGS. 3A and 3B, the catheter body 12 comprises an elongated tubular construction having a single, axial or central lumen 26. The catheter body 12 is flexible, i.e., bendable, but substantially non-compressible along its length. The catheter body 12 can be of any suitable construction and made of any suitable material. One construction comprises an outer wall 27 made of polyurethane or PEBAX. The outer wall 27 comprises an embedded braided mesh of stainless steel or the like to increase torsional stiffness of the catheter body 12 so that, when the control handle 16 is rotated, length of the catheter rotates in a corresponding manner.

The outer diameter of the catheter body 12 is not critical. Likewise the thickness of the outer wall 27 is not critical, but is thin enough so that the central lumen 26 can accommodate lead wires, and any other desired wires, cables or tubings. If desired, the inner surface of the outer wall 27 is lined with a stiffening tube 28 to provide improved torsional stability.

Components extending through the central lumen 26 of the catheter body 12 include at least an irrigation tubing 36 to supply fluid to the ring electrodes 19 on the distal electrode assembly 18, thermocouple wires 46 and 48 for measuring temperature, for example, at the ring electrodes 19, lead wires 40 for the ring electrodes 19, puller wire 35 and its compression coil 37, the extender 60 for the anchoring assembly 22, and a cable 50 for an electromagnetic sensor 52 located at the distal end of the distal section 17.

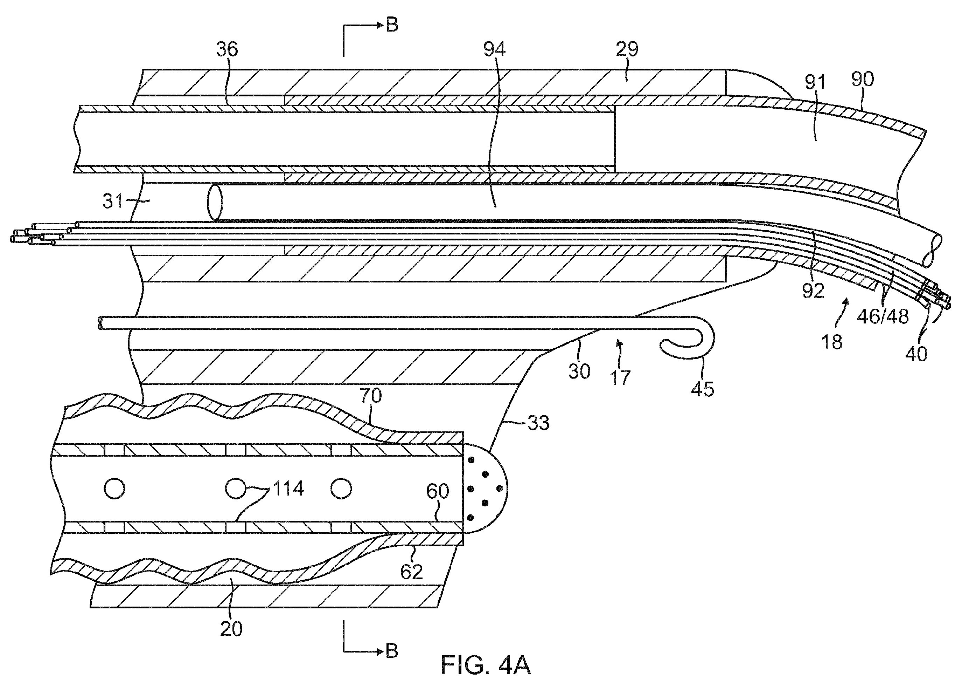

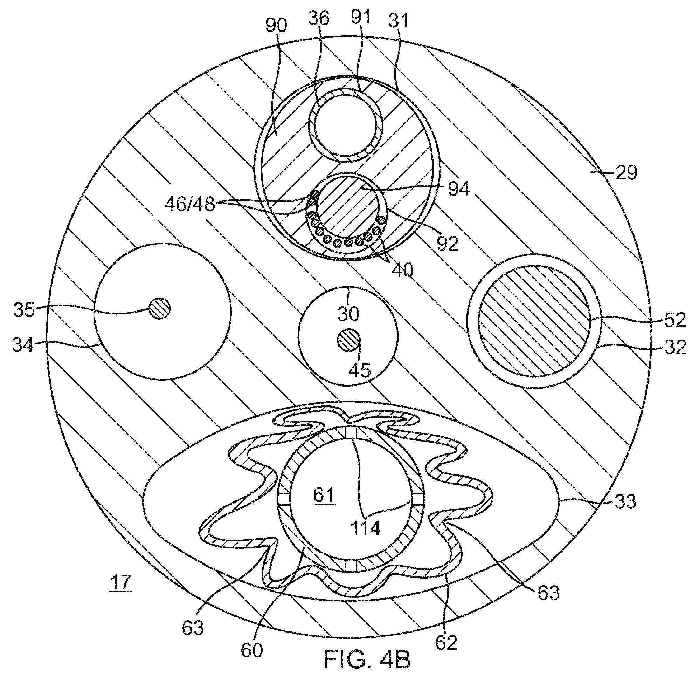

With reference to FIGS. 4A and 4B, the distal section 17 comprises a section of multi-lumened tubing 29, with on-axis and off-axis lumens. In a disclosed embodiment, the tubing 29 has at least on-axis lumen 30 and off-axis lumens 31, 32 and 33. The on-axis lumen 30 is configured to pass a guide wire 45. The first off-axis lumen 31 is configured for the lead wires 40 , the irrigation tubing 36 and the thermocouple wires 46 and 48. The second off-axis lumen 32 is configured for the cable 50. The third off-axis lumen 33 is configured for stowing the balloon member 62 mounted on a distal portion of the extender 60. A fifth off-axis lumen 34 is configured for the puller wire 35.

The tubing 29 of the distal section 17 is made of a suitable non-toxic material that is preferably more flexible than the catheter body 12. A suitable material for the tubing 29 is braided PEBAX or polyurethane, i.e., polyurethane with an embedded mesh of braided stainless steel or the like. The size of each lumen is not critical, but is sufficient to house the respective components extending therethrough.

The useful length of the catheter, i.e., that portion that can be inserted into the body excluding the assemblies 18 and 22, can vary as desired. In some embodiments, the useful length ranges from about 110 cm to about 120 cm. The length of the distal section 17 is a relatively small portion of the useful length, and preferably ranges from about 3.5 cm to about 10 cm, more preferably about 4 cm to about 8 cm, and still more preferably about 6.5 cm.

In some embodiments as illustrated in FIG. 3A, the proximal end of the tubing 29 of the distal section 17 is received in a distal end of the catheter body 12. An outer surface of the tubing 29 faces an inner surface of the outer wall 27 of the catheter body 12. The junction between the catheter body 12 and the distal section 17 is sealed by an adhesive 24, e. g., epoxy.

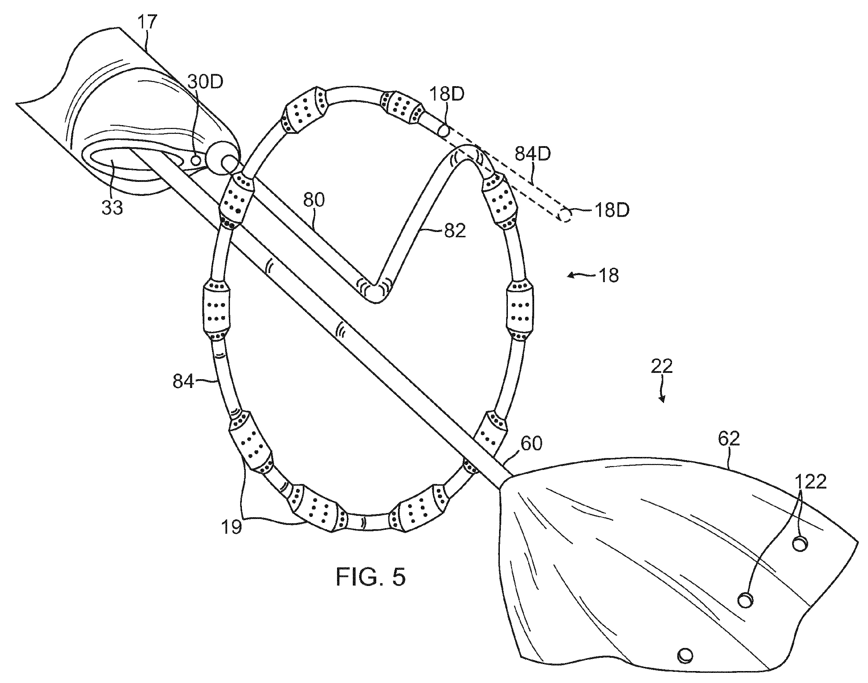

At the distal end of the tubing 27 of the distal section 17 is the electrode assembly 18. In some embodiments, the assembly 18 is adapted to sit on the ostium 21 of the tubular region 25 and the anchoring assembly 22 is adapted to enter the tubular region, as shown in FIG. 2, and contact the inner surface 23 of the tubular region 25. With reference to FIG. 5, the electrode assembly 18 is generally concentric about the longitudinal axis of the catheter and includes a generally on-axis proximal axial linear segment 80, a radial linear segment 82 and a generally circular distal main segment 84. The ring electrodes 19 are carried on the generally circular main segment 84 which extends about 360 degrees about the longitudinal axis of the catheter. The generally circular main segment 84 may include a distal linear segment 84D (shown in broken lines in FIG. 5) which may facilitate entry of the circular segment 84 into a guiding sheath (not shown).

The generally circular main segment 84 is generally traverse to the catheter body 12, for example, generally perpendicular to the catheter body 12. The generally circular main segment 84 need not form a flat circle, but can be very slightly helical. The generally circular main segment 84 has an exposed length ranging between about 40mm and 100mm, more preferably about 50mm and 90mm, and still more preferably about 60mm, and an outer diameter preferably ranging to about 10 mm to about 35 mm, more preferably about 15 mm to about 30 mm, still more preferably about 25 mm. The generally circular main segment 84 can curve in a clockwise direction, as shown in FIG. 5, or in a counterclockwise direction.

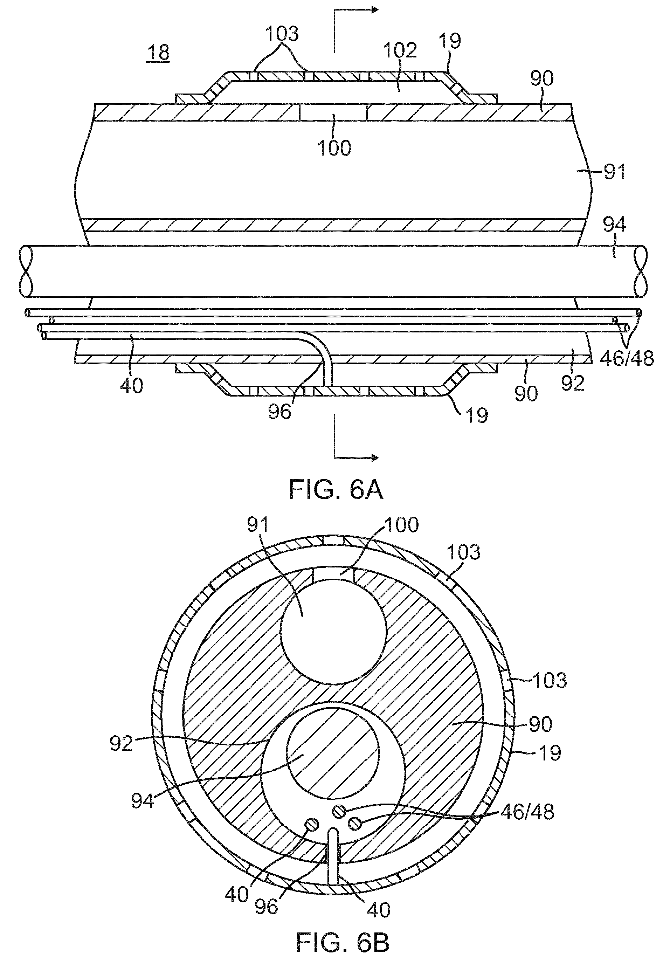

In some embodiments, as shown in FIGS. 4A, 6A and 6B, the electrode assembly 18 comprises a multi-lumened tubing 90 with at least a first lumen 91 and a second lumen 92. The first lumen 91 is configured to pass irrigation fluid to the ring electrodes 19. The second lumen 92 is occupied by the lead wires 40, at least one thermocouple wire pair 46 and 48, and a support member 94 providing shape and support to the electrode assembly 18. Proximal ends of the tubing 90 and the support member 94 extend a short distance into the distal end of the lumen 31 of the distal section 17 to anchor the electrode assembly 18. The tubing 90 of the electrode assembly 18 can be made of any suitable material that is flexible and biocompatible and preferably plastic, such as polyurethane or PEBAX. The support member 94 has shape memory in that it can be straightened or bent out of its original shape upon exertion of a force and is capable of substantially returning to its original shape upon removal of the force. A suitable material for the shape memory support member 94 is a nickel/titanium alloy. Such alloys typically comprise about 55% nickel and 45% titanium, but may comprise from about 54% to about 57% nickel with the balance being titanium. A preferred nickel/titanium alloy is nitinol, which has excellent shape memory, together with ductility, strength, corrosion resistance, electrical resistivity and temperature stability.

The lead wires 40 for the ring electrodes 19 extend through the lumen 92 of the tubing 90. In the generally circular main segment 84, each lead wire is connected to a respective ring electrode 19, for example, in the manner illustrated in FIG. 6A, through a hole 96 formed in the sidewall of the tubing 90. The support member 94 also extending through the lumen 92 has a distal end anchored in the distal end of the tubing 90 which may be sealed by suitable material, such as epoxy to form an atraumatic dome end 18D (see FIG. 5). A short ring (not shown), made of metal or plastic, and preferably polyamide, may be mounted in the distal end of the tubing 90 to prevent the distal end from collapsing.

As shown in FIG. 4A, a distal end of the irrigation tubing 36 extends a short distance into the proximal end of the lumen 91 of the tubing 90 of the electrode assembly 18. As shown in FIG. 6A, fluid delivered by the irrigation tubing 36 passes into the lumen 91 and irrigates the ring electrodes 19 via holes 100 formed in the sidewall of the tubing 90. The fluid exits the lumen 91 and enters an annular gap 102 defined by each ring electrode 19 circumferentially around the tubing 90 and exits the ring electrode 19 via holes 103 formed in the sidewall of the ring electrode 19.

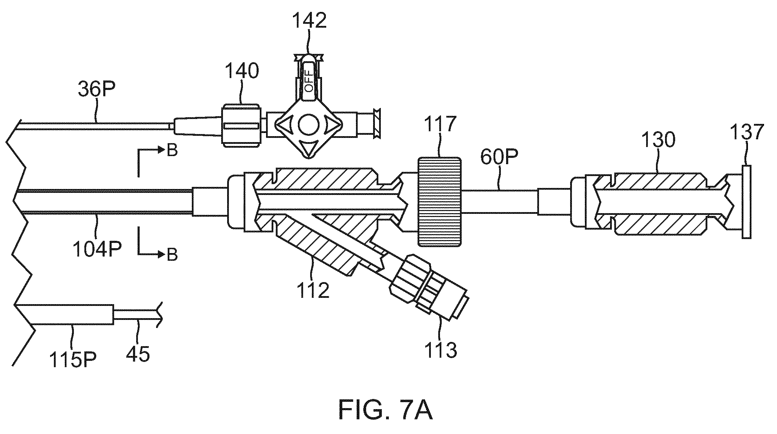

The irrigation tubing 36 which delivers fluid for the ring electrodes 19 extends through the lumen 31 of the distal section 17, and the central lumen 26 of the catheter body 12. A proximal portion 36P may extend as a side arm 27 of the control handle 16, as shown in FIG. 1, or it may extend proximally of the control handle 16, as shown in FIG. 7A. In any case, the irrigation tubing 36 terminates in a valve adapter 140 with a stopcock 141 for connection with an irrigation fluid source (not shown).

As mentioned above, the lumen 33 provides a nesting site 20 (FIG. 3A) in the distal section 17 housing the balloon member 62 of the anchoring assembly 22. The extender 60 is afforded longitudinal movement relative to the catheter for advancing the balloon member 62 from the lumen 33 for deployment and drawing the balloon member 62 back into the lumen 33 for stowage. In the illustrated embodiment of FIGS. 1 and 7A, the extender 60 has a proximal portion 60P that is proximal of the control handle 16 and exposed for manipulation by an operator. The extender 60 passes through the control handle 16, the catheter body 12, and the lumen 33 of the distal section 17. A distal portion 60D supports and extends through the balloon member 62.

In the stowed position inside the lumen 33, the balloon member 62 is deflated and collapsed, as shown in FIG. 4B. In that regard, preformed folds or pleats 63 may be provided in the balloon material to facilitate stowage. When deployed by the extender 60, the balloon member 62, as shown in FIGS. 4 and 5, is distal of the electrode assembly 18 by an extended distance and inflated to attain a desirable circumference for contacting the inner surface 23 of the tubular region 25 and releasably wedging the balloon member 62 in the tubular region 25, as shown in FIG. 2.

The balloon member 62 includes a balloon membrane 70 constructed of any suitable material. The material may be of the highly compliant variety, such that the material is elastically flexible and stretches upon application of pressure. Suitable materials include elastomers, such as, for example, silicone, latex, and low durometer polyurethane (for example, a durometer of about 80A). In the illustrated embodiment, the balloon membrane 70 has distal and proximal ends which are affixed to the extender 60 to form fluid tight seals. However it is understood that the balloon membrane may be configured otherwise as known in the art.

To inflate the balloon member 62, inflation fluid is passed through a lumen 61 of the extender 60 of the anchoring assembly 22, as shown in FIGS. 3A. In some embodiments, as shown in FIG. 7A, the proximal end 60P of the extender 60 extends proximally past the control handle 16, where it is connected to a hemostasis valve 130 with a proximal end 137 (for example, a female luer lock) that is adapted to receive inflation fluid from an inflation fluid source (not shown). The inflation fluid travels through the lumen 61 of the extender 60 and exits the lumen 61 via ports 114 formed in the side wall of the distal portion of the extender 60 inside the balloon member 62, as shown in FIGS. 2 and 4A. As shown in FIGS. 2 and 5, ports 122 are formed in the balloon membrane 70 to allow inflation fluid to exit the balloon for irrigating and cooling the tissue of the tubular region 25 in which the balloon member 62 is anchored.

To deliver fluid into the lumen 33, for example, to flush out the lumen 33 and/or lubricate the balloon member 62 in preparation for deployment or stowage, a fluid tubing 104 is provided to surround the extender 60 and extend coaxially therewith for most of its length, including part of the proximal portion 60P. In some embodiments, the tubing 104 has a distal end terminating within the lumen 33 of the tubing 29 of the distal section 17 and an outer diameter size to form a fluid tight seal with the lumen 33. Its own lumen 105 is sized to provide an annular gap around the outer surface of the extender 60 to allow fluid to pass alongside (FIGS. 3A and 3B). In some embodiments, as illustrated in FIG. 7A, the tubing 104 has a proximal end 104P terminating outside of the control handle 16, on which a hemostasis valve 112, e.g., a Tuohy Borst valve with a side arm 113, is mounted for introducing lubricating fluid into the tubing 104 and the lumen 33 from a lubricating fluid source (not shown).

The hemostasis valve 112 is distal of the hemostasis valve 130 so that the extender 60 can pass through the lumen 105 of the fluid tubing 104 and extend coaxially therewith. As a Tuohy-Borst, for example, the hemostasis valve 112 has a gripping member 117 which upon rotation can releasably lock the extender 60 in a fixed position to prevent longitudinal movement of the extender 60 relative to the catheter.

A guide wire tubing or "inner body" 115 with lumen 116 is provided to allow a guide wire 45 (FIG. 4A) to pass through the catheter and help advance the catheter through a patient's vasculature. The guide wire inner body 115 has an exposed portion 115P proximal of the control handle 16, as shown in FIGS. 1 and 7A, allowing an operator access to the lumen 116 for insertion of the guide wire 45 at a proximal end of the inner body 115P. The exposed proximal portion 115P may extend as a side arm of the control handle 16 as shown in FIG. 1, or it may extend proximally from the control handle as shown in FIG. 7A. In any case, the tubing 115 extends distally from the control handle 16, through the lumen 26 of the catheter body 12 (FIG. 3A) and may terminate anywhere along the lumen 30 of the tubing 29 (FIG. 3A), which has a distal opening 30D communicating with outside the distal section 17 of the catheter (FIG. 5). In these embodiments, the guide wire inner body 115 extends longitudinally along the catheter, separately from the extender 60.

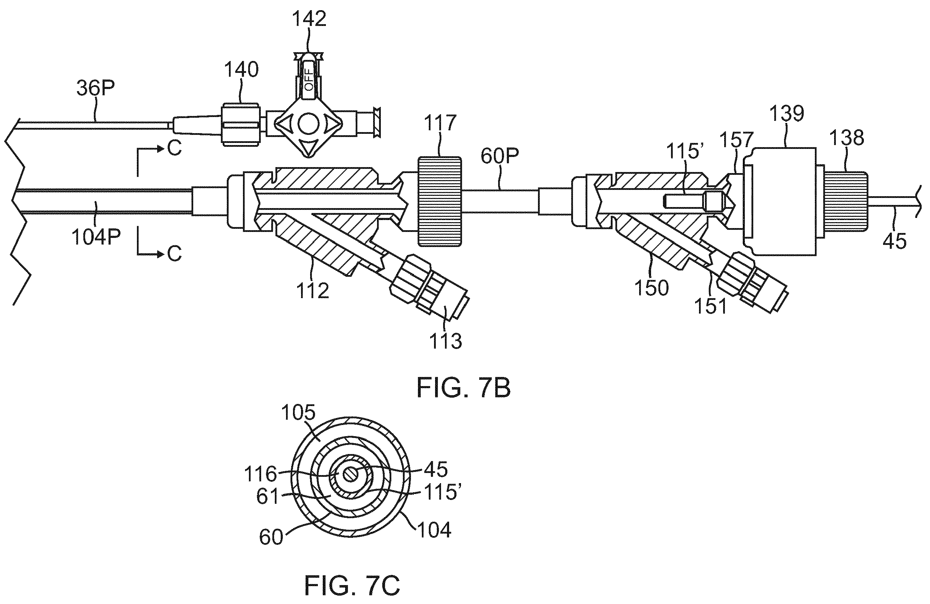

However, in other embodiments, as illustrated in FIGS. 7B and 7C, guide wire inner body 115' is coaxial with the extender 60, extending through the lumen 61 of the extender 60. The guide wire inner body 115' extends distally from a hemostasis valve 150 mounted on a proximal end of the extender 60P. The valve 150 has a side arm 151 for receiving the balloon member inflation fluid from a fluid source (not shown). The valve 150 also has a female luer lock 147 connected to another hemostasis valve 139, e.g., a Tuohy Borst, with a gripping member 138. The inner body 115' has a proximal end distal of the gripping member 138 so that the portion of the guide wire 45 proximal of the proximal end of the inner body 115' is gripped by the gipping member 138.

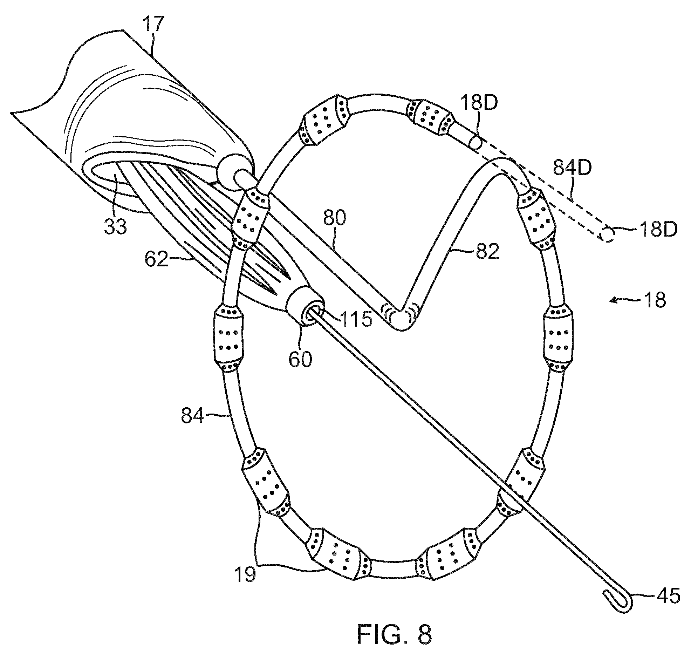

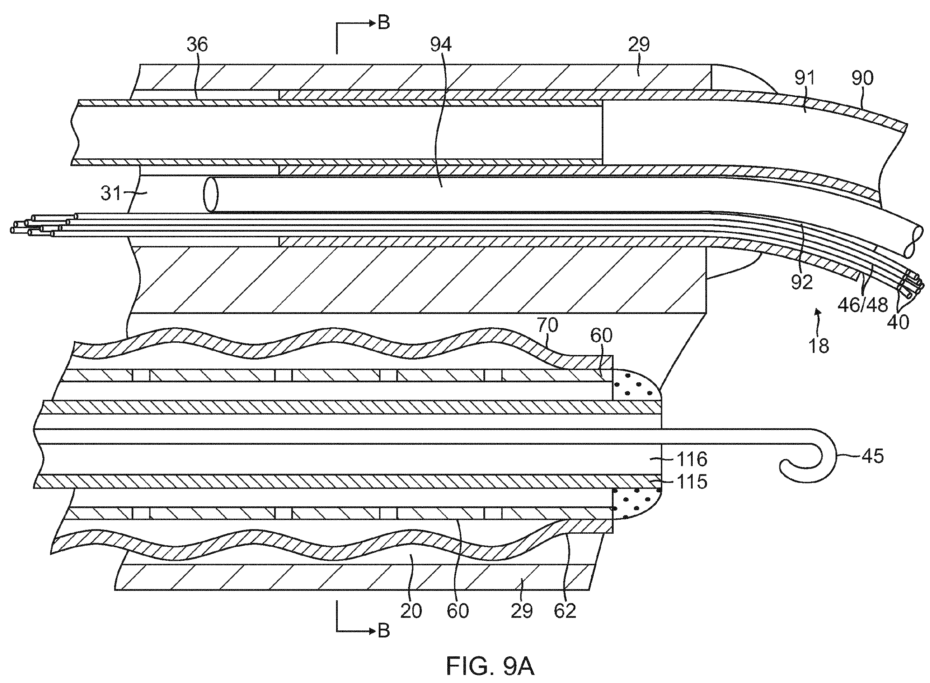

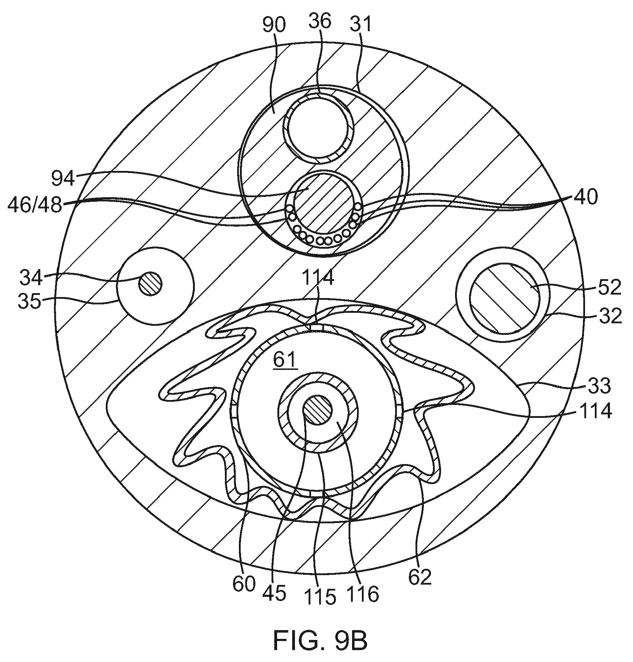

It is understood that the guide wire inner body 115' may have a distal end terminating in the valve 150 or anywhere distally in the lumen 61 of the extender 60. In some embodiments, as shown in FIG. 8, the inner body 115' extends through the balloon member 60, within the lumen 61 of the extender 60, with a distal end 115D that exits the lumen 61 at a distal end of the balloon member 60. In these embodiments, the tubing 29 of the distal section 17 has four lumens, as shown in FIGS. 9A and 9B. Lumen 31 carries the irrigation tubing 36, the thermocouple wire pair 46 and 48, the lead wires 40. Lumen 32 carries the sensor 52 and the sensor cable 50. Lumen 33 carries the balloon member 62, the extender 60, and the guide wire tubing 115 in the lumen 61. Lumen 34 carries the puller wire. A distal portion of the lumen 32 receives a proximal end of the tubing 90 of the distal electrode assembly 18 and the support member 94.

In any embodiment, the distal section 17 can be rendered deflectable by means of the puller wire 35 whose proximal end is anchored in the control handle 16 and whose distal end is anchored in a side wall of the tubing 29 near a distal end of the tubing 29. The puller wire 35 extends through the catheter body 12 as shown in FIGS. 3A and 3B, and through the lumen 34 of the distal section 17, as shown in FIG. 4B. Throughout the length of the catheter body 12, the puller wire 35 is surrounded by a compression coil 37, as known in the art, to enable deflection of the portion of the catheter distal of a distal end of the compression coil 37. As also known in the art, a distal end of the puller wire may be anchored, for example, by means of a T-shaped anchor in the sidewall of the tubing 29. The puller wire 35 can made of any suitable metal, such as stainless steel or Nitinol, and is preferably coated with Teflon.RTM. or the like. The coating imparts lubricity to the puller wire. The puller wire preferably has a diameter ranging from about 0.006 to about 0.010 inch. The portion of the puller wire 35 extending through the lumen 34 of the distal section 17 may have a TEFLON coating to prevent the puller wire from cutting into the sidewall of the tubing 29.

Each of the ring electrodes 19 is electrically connected to an appropriate mapping or monitoring system and/or source of ablation energy via the lead wires 40 whose proximal ends are connected to an electrical connector 120 at the proximal end of the control handle 16 (FIG. 1). Throughout the catheter, the lead wires 40 may be enclosed within a protective, nonconductive sheath (not shown), which can be made of any suitable material, preferably polyimide.

The ring electrodes can be made of any suitable solid conductive material, such as platinum or gold, preferably a combination of platinum and iridium, and mounted onto the tubing with glue or the like. Alternatively, the ring electrodes can be formed by coating the tubing with an electrically conducting material, like platinum, gold and/or iridium. The coating can be applied using sputtering, ion beam deposition or an equivalent technique. While the electrode assembly 18 is illustrated with ring electrodes separated at equal distance from adjacent ring electrodes configured for uni-polarity sensing it is understood that the ring electrodes may be configured for bi-polarity sensing in paired configuration on the assembly 18.

The number of the ring electrodes 19 on the electrode assembly 18 can vary as desired. The number of ring electrodes ranges from about six to about twenty, preferably from about eight to about twelve. Where any of the ring electrodes 19 are adapted for ablation, a pair of thermocouple wires 46 and 48 can be provided to detect temperature of a respective ring electrode. The thermocouple wires 46 and 48 extend through the catheter body 12 (FIGS. 3B and 4A) and into the lumen 31 of the tubing 29 of the distal section 17 and further into the lumen 91 of the tubing 90 of the electrode assembly 18. (FIG. 4A).

The compression coil 37 is made of any suitable metal, preferably stainless steel, and is tightly wound on itself to provide flexibility, i.e., bending, but to resist compression. The inner diameter of the compression coil is preferably slightly larger than the diameter of the puller wire 35. A Teflon.RTM. coating on the puller wire 35 allows it to slide freely within the compression coil. Within the catheter body 12, the outer surface of the compression coil 37 is also covered by a flexible, non-conductive sheath (not shown), e.g., made of polyimide tubing. The compression coil is anchored at its proximal end to the catheter body 12 near a proximal end of the catheter body.

In use, a guiding sheath is inserted in the patient's body and advanced through the patient's vasculature to reach a target region of the heart, for example, the left atrium. The guide wire 45 is then inserted into the guide wire tubing 115 at its proximal end outside of the control handle 16, through the catheter, distally past the opening 30D of the distal section 17 (FIG. 5) or the distal end of the extender 60 (FIG. 8), and into the patient's vasculature via the guiding sheath to reach the target region. According to some embodiments, the anchoring assembly 22 is deployed from the distal section 17 before the catheter enters the patient's body. For example, prior to deployment of the anchoring assembly 22, fluid is passed into the tubing 104 via the side arm 113 of the valve 112 to flush out the lumen 33 in which the balloon member 62 of the anchoring assembly 22 is nested. After flushing, the exposed portion 60P of the extender 60 is advanced the extender relative to the control handle 16 and deploy the balloon member 62 from the lumen 33. The balloon member 62 (in the collapsed configuration) is advanced past the distal electrode assembly 18, for example, passed through an open region 85 (FIG. 5) circumscribed by the generally circular main portion 84. The operator then inserts the balloon member 62 into the guiding sheath and advances it through the patient's vasculature via the proximal extender portion 60P until the balloon member 62t passes a distal end of the guiding sheath and reaches the target region. The balloon member 62 is then advanced into a tubular region of the left atrium, for example, a pulmonary vein, where the operator inflates with fluid that is delivered into the balloon member via the lumen 61 of the extender 60 which receives inflation fluid via the valve adapter 130 of FIG. 7A, or the side arm 151 of the valve 150 in FIG. 7B. With sufficient inflation, the balloon member 62 is appropriately lodged in the tubular region 25 as an anchor for the electrode assembly 18.

Before or after the balloon member 62 is inflated and lodged, the operator inserts the distal end of the electrode assembly 18 into the guiding sheath to enter the patient's vasculature, by straightening the distal electrode assembly 18. The operator advances the electrode assembly 18, followed by the distal section 17 and the catheter body 12, through the guiding sheath, as guided by the guide wire 45, and also by the extender 60 over which the catheter body passes. When the electrode assembly 18 reaches the target region and passes the distal end of the guiding sheath, the operator positions the electrode assembly 18 to sit on the ostium 21. The operator may secure the position of the electrode assembly 18 relative to the balloon member 62 by locking the position of the extender 60 relative to the control handle 16. The electrode assembly 18 is then stabilized on the ostium by the extender 60 and the balloon member 62. As shown in FIG. 2, the extender 60 extends through the open region 85 circumscribed by the generally circular main portion 84 of the electrode assembly 18 and thus the anchoring assembly 22 provides both axial and radial stabilization to the electrode assembly 18.

When the operator has arranged the balloon member 62 and the electrode assembly 18 in position for securing the assembly 18 on the ostium, the operator can actuate the gripping member 117 of the valve 112 to lock the extender 60 relative to the fluid tubing 104 which is fixed to the control handle 16 and the catheter. The gripping member 117 locks the extender 60 to the catheter which in turn locks the balloon member 62 and the electrode assembly 18 to each other so that the balloon member 62 anchors the electrode assembly 18. This locking action frees up the need for the operator or an assistant to hold and press the electrode assembly 18 on the ostium.

When the operator wants to relocate the distal electrode assembly 18, for example, to another pulmonary vein and ostium, the operator may stop the flow of inflation fluid to the balloon member 62 which then deflates via the ports 122 in the balloon membrane 70 sufficiently for the balloon member 62 to be dislodged from the tubular region 25. The operator releases the gripping member 117 and draws the proximal extender portion 60P to retract the balloon member 62 closer toward the electrode assembly 18 or to restow it back into the lumen 33. The operator repositions the balloon member 62 into a second tubular region by manipulating the extender portion 60 to advance the balloon member 62 into the second tubular region. The operator then inflates the balloon member 62 to lodge the balloon member 62, and locks the gripping member 117 so that the balloon member 62 anchors the electrode assembly 18 on the ostium of the second tubular region.

After diagnostic and/or therapeutic treatment of the pulmonary veins of the left atrium, the operator deflates the balloon member 62, draws the balloon member back into the lumen 33, draws the catheter back into the guiding sheath, and removes the catheter from the patient's body.

The preceding description has been presented with reference to certain exemplary embodiments of the invention. Workers skilled in the art and technology to which this invention pertains will appreciate that alterations and changes to the described structure may be practiced without meaningfully departing from the principal, spirit and scope of this invention, and that the drawings are not necessarily to scale. Moreover, it is understood that any one feature of an embodiment may be used in lieu of or in addition to feature(s) of other embodiments. Accordingly, the foregoing description should not be read as pertaining only to the precise structures described and illustrated in the accompanying drawings. Rather, it should be read as consistent with and as support for the following claims which are to have their fullest and fairest scope.

## Claims

1. A catheter comprising:
an elongated catheter body;
a distal section including a multi-lumened tubing distal of the catheter body, including at least a first lumen and a second lumen;
an electrode assembly distal of the distal section, a proximal end of the electrode assembly received in the first lumen; and
a stabilizing assembly movable between a stowed position and a deploy positioned, the stabilizing assembly having a balloon member that is housed in the second lumen of the distal section when the stabilizing assembly is in the stowed position and is distal of the electrode assembly when the stabilizing assembly is in the deployed position.

2. The catheter of claim 1, wherein the stabilizing assembly includes an extender having a distal portion on which the balloon member is mounted, the extender being longitudinally movable relative to at least the distal section.

3. The catheter of claim 2, wherein the extender extends longitudinally through the catheter body and the distal section, or wherein the extender has a distal portion extending through an interior of the balloon member, or wherein the extender has a lumen therethrough along its length.

4. The catheter of claim 3 wherein the extender has a lumen therethrough along its length, wherein the distal portion of the extender has ports configured to allow fluid to pass from the lumen of the extender to the interior of the balloon member to inflate the balloon.

5. The catheter of claim 3 wherein the extender has a lumen therethrough along its length, further comprising a guide wiretubing

6. The catheter of claim 5, wherein the guide wire tubing extends through the lumen of the extender.

7. The catheter of claim 1, wherein the electrode assembly has a generally circular main portion configured to contact an ostium.

8. The catheter of claim 7, wherein the balloon member is configured to be wedged in a tubular region of the ostium when the balloon member is inflated.

9. A catheter adapted for use in or near a tubular region of the heart, comprising:
an elongated catheter body;
a distal section including a multi-lumened tubing distal of the catheter body, including at least a first lumen and a second lumen;
an electrode assembly distal of the distal section, the electrode assembly having a generally circular main portion configured to contact an ostium of the tubular region, the generally circular main portion having at least one ring electrode;
a balloon assembly longitudinally movable relative to the distal section between a stowed position and a deployed position, wherein the balloon is housed in the second lumen in the stowed position, and the balloon is advanced into the tubular region distally of the electrode assembly in the deployed position to stabilize the electrode assembly on the ostium.

10. The catheter of claim 9, wherein the balloon assembly includes an elongated extender and a balloon member mounted on a distal portion of the extender.

11. The catheter of claim 12, wherein the extender extends along the length of the catheter and has a proximal portion adapted for manipulation by a user to move the balloon between the stowed position and the deployed position, or wherein the extender has a lumen therethough to pass fluid to inflate the balloon, or wherein the extender has a sidewall with at least one port through which fluid passes into the balloon.

12. The catheter of claim 10, wherein the balloon assembly includes a fluid tubing surrounding the extender, the fluid tubing having a lumen to pass fluid to flush the balloon assembly when stowed in the second lumen.

13. The catheter of claim 9, further comprising a guide wire tubing extending through the catheter.

14. The catheter of claim 13, wherein the guide wire tubing extends through the balloon assembly.

15. The catheter of claim 10, further comprising a guide wire tubing that extends through a lumen of the extender.

16. The catheter of claim 14, wherein at least the guide wire tubing and the extender are coaxial.
